# EUROPEAN PATENT APPLICATION

(11) **EP 4 385 546 A2**
(43) Date of publication of application: **19.06.2024**
(21) Application number: 24174147.9
(22) Date of filing: 13.03.2020
(51) Int. Cl.: A61M 11/04

(54) **SMOKING SUBSTITUTE SYSTEM**

(30) Priority: 22.03.2019 EP 19020208
(62) Divisional of application: 20715718.1
(71) Applicant: Imperial Tobacco Limited, Bristol, BS3 2LL (GB)
(72) Inventor: Benyezzar, Med, Bristol, BS3 2LL (GB); Zitzke, Roland, Bristol, BS3 2LL (GB)
(74) Representative: Mewburn Ellis LLP

(57) **Abstract**

A heat-not-burn device is described. The device includes a controller having a cyclic operating routine. The controller is configured to, during each cycle of the cyclic operating routine, perform at least one function of the device. A period of the cyclic operating routine is between 10 milliseconds and 500 milliseconds.

## Description

### TECHNICAL FIELD

The present invention relates to a smoking substitute system and particularly, although not exclusively, to a smoking substitute system comprising a heat-not-burn device configured to ensure safe operation of the device.

### BACKGROUND

The smoking of tobacco is generally considered to expose a smoker to potentially harmful substances. It is generally thought that a significant amount of the potentially harmful substances are generated through the heat caused by the burning and/or combustion of the tobacco and the constituents of the burnt tobacco in the tobacco smoke itself.

Conventional combustible smoking articles, such as cigarettes, typically comprise a cylindrical rod of tobacco comprising shreds of tobacco which is surrounded by a wrapper, and usually also a cylindrical filter axially aligned in an abutting relationship with the wrapped tobacco rod. The filter typically comprises a filtration material which is circumscribed by a plug wrap. The wrapped tobacco rod and the filter are joined together by a wrapped band of tipping paper that circumscribes the entire length of the filter and an adjacent portion of the wrapped tobacco rod. A conventional cigarette of this type is used by lighting the end opposite to the filter, and burning the tobacco rod. The smoker receives mainstream smoke into their mouth by drawing on the mouth end or filter end of the cigarette.

Combustion of organic material such as tobacco is known to produce tar and other potentially harmful by-products. There have been proposed various smoking substitute systems (or "substitute smoking systems") in order to avoid the smoking of tobacco.

Such smoking substitute systems can form part of nicotine replacement therapies aimed at people who wish to stop smoking and overcome a dependence on nicotine.

Smoking substitute systems include electronic systems that permit a user to simulate the act of smoking by producing an aerosol (also referred to as a "vapour") that is drawn into the lungs through the mouth (inhaled) and then exhaled. The inhaled aerosol typically bears nicotine and/or flavourings without, or with fewer of, the odour and health risks associated with traditional smoking.

In general, smoking substitute systems are intended to provide a substitute for the rituals of smoking, whilst providing the user with a similar experience and satisfaction to those experienced with traditional smoking and with combustible tobacco products. Some smoking substitute systems use smoking substitute articles (also referred to as a "consumables") that are designed to resemble a traditional cigarette and are cylindrical in form with a mouthpiece at one end.

The popularity and use of smoking substitute systems has grown rapidly in the past few years. Although originally marketed as an aid to assist habitual smokers wishing to quit tobacco smoking, consumers are increasingly viewing smoking substitute systems as desirable lifestyle accessories.

There are a number of different categories of smoking substitute systems, each utilising a different smoking substitute approach.

One approach for a smoking substitute system is the so-called Heated Tobacco ("HT") approach in which tobacco (rather than an "e-liquid") is heated or warmed to release vapour. HT is also known as "heat not burn" ("HNB"). The tobacco may be leaf tobacco or reconstituted tobacco. The vapour may contain nicotine and/or flavourings. In the HT approach the intention is that the tobacco is heated but not burned, i.e. the tobacco does not undergo combustion.

A typical HT smoking substitute system may include a device and a consumable. The consumable may include the tobacco material. The device and consumable may be configured to be physically coupled together. In use, heat may be imparted to the tobacco material by a heating element of the device, wherein airflow through the tobacco material causes components in the tobacco material to be released as vapour. A vapour may also be formed from a carrier in the tobacco material (this carrier may for example include propylene glycol and/or vegetable glycerine) and additionally volatile compounds released from the tobacco. The released vapour may be entrained in the airflow drawn through the tobacco.

As the vapour passes through the consumable (entrained in the airflow) from the location of vaporisation to an outlet of the consumable (e.g. a mouthpiece), the vapour cools and condenses to form an aerosol for inhalation by the user. The aerosol will normally contain the volatile compounds.

In HT smoking substitute systems, heating as opposed to burning the tobacco material is believed to cause fewer, or smaller quantities, of the more harmful compounds ordinarily produced during smoking. Consequently, the HT approach may reduce the odour and/or health risks that can arise through the burning, combustion and pyrolytic degradation of tobacco.

There may be a need for improved design of smoking substitute systems, in particular HT smoking substitute systems, to enhance the user experience and improve the function of the HT smoking substitute system.

The present disclosure has been devised in the light of the above considerations.

### SUMMARY OF THE INVENTION

At its most general, the present invention relates to the inclusion of a controller in the heat-not-burn device configured to run a cyclic operating routine. This may permit improved computational stability of the controller

According to a first aspect of the present invention, there is provided a heat not burn device including a controller having a cyclic operating routine, wherein the controller is configured to, during each cycle of the cyclic operating routine, perform at least one function of the device; and wherein a period of the cyclic operating routine is between 10 milliseconds and 500 milliseconds.

By providing a device according to the first aspect, the device has the ability to ensure safe operations and increased stability. In one example, the controller may be configured to detect the temperature of the heater, at continuous intervals, during each cycle of the cyclic operating routine to ensure safe operation of the device. In some embodiments, making measurement during each cycle permit the controller more time to dedicate other running processes which may check the device safety.

Optional features will now be set out. These are applicable singly or in any combination with any aspect.

Optionally, said period of the cyclic operating routine is between 150 milliseconds and 400 milliseconds.

Optionally, at least one of the at least one function is performed exactly once during each cycle of the cyclic operating routine.

Optionally, each of the at least one function is performed exactly once during each cycle of the cyclic operating routine.

Optionally, the at least one function includes measuring a heater temperature of a heater of the device.

Optionally, the at least one function includes comparing the heater temperature with a predetermined heater threshold.

Optionally, the at least one function includes controlling a power supplied to the heater based on the comparison between the heater temperature and the predetermined heater threshold.

Optionally, the device further comprises a voltage detection means connected to the controller, and the at least one function including measuring an output voltage of a power supply of the device.

Optionally, the at least one function includes controlling a power supplied to the heater based on the measured output voltage.

Optionally, the device further comprises an ambient temperature sensor connected to the controller and wherein the at least one function includes measuring an ambient temperature for the device.

Optionally, the at least one function includes controlling a power supplied to the heater based on the measured ambient temperature.

Optionally, the at least one function includes making a determination of the presence of a user input command to an input of a user interface of the device.

Optionally, the at least one function includes updating an output of a user interface of the device.

Optionally, the at least one function includes determining the puff status of a puff sensing means of the device.

Optionally, the at least one function includes making a determination of a charging status of a power supply of the device.

Optionally, the controller includes a microcontroller or a microprocessor.

In some embodiments, the controller may be configured to measure the temperature of a heater during each cycle of the cyclic operating routine. To achieve this, the controller may remain connected to a temperature sensor which is configured to constantly measure the temperature of the heater and simultaneously indicate the same to the controller. The frequency of measuring temperature by the sensor may correspond to the period of cyclic routine check selected by the controller. In one example, if the period of cyclic routine check is selected to be small the sensor may be configured to measure and share the temperature of the heater, with the controller, less frequently. Whereas, when the period of cyclic routine check is selected to be comparatively long, the sensor may be configured to measure and share the temperature of the heater, with the controller, more frequently.

Advantageously, the controller may be configured to compare the temperature of the heater with a predetermined threshold. The predetermined threshold may denote a cut-off value of temperature above which the heater shall not be heated. Said value may be pre-stored in the memory of the device. In some embodiments, the predetermined threshold may depend on a present operating mode of the device. The controller may be configured to always compare the measured value of temperature with this predetermined threshold. This ensures that the heater of the device does not overheat, thus preventing any damage to be caused to the heater or to the consumable.

Conveniently, the controller may be configured to control the power supply to the heater based on the comparison between the measured temperature and the predetermined threshold. In an embodiment, this ensures the safety of the device and the consumable. For example, if the temperature of the heater is determined to be above the threshold, the controller may disconnect the supply of power from the power source to the heater.

Optionally, the device may be configured to have a voltage regulator to ensure that the correct amount of voltage is supplied to the heater during the cyclic operating routine. In one example, the voltage regulator may remain connected to the controller. Further, the controller may be configured to calculate the amount of voltage to be supplied to the heater based on the current temperature of the heater and the mode selected. This information may then be passed to the voltage regulator to control voltage supply to the heater. In some embodiments, power supplied to heater may be controlled via pulse width modulation of the output voltage of the power supply.

In some embodiments, the device may include a temperature sensor to measure the ambient temperature. Said temperature sensor may be connected to the controller to allow the controller to decide when to disable the heater of the device and/or a particular mode of operation. To achieve this, the temperature sensor measures the ambient temperature and passes this information to the controller. The controller may be able to take various decision based on this information. For example, the controller may be able to take a decision to disable the heater in response to detecting that the ambient temperature is too high. Further, the controller may be able to take a decision one or more operational modes of the device in response to detecting that the ambient temperature is too high.

In some embodiments, the device may include a plurality of output means to provide status update of the device to the user. The output means may be connected to the controller such that upon receiving signal from the controller, the output device may indicate the status update of the device. In one example, the output means may include a plurality of LEDs that may be controlled by the controller to indicate disabling of heater, change of mode and status. In other examples, the output means may be configured to provide audio, visual or haptic feedback indicating the status of the device.

Conveniently, the device may include a plurality of input means connected to the controller and configured to receive user input related to various operations of the device. In some embodiment, the input means may include a button which is configured to receive a user input in response to pressing. Further, the input means may include a microphone, a motion sensor etc.

In some embodiment, the device may comprise a hall sensor connected to the controller. The hall sensor may be configured to detect one of insertion and removal of a cap of the device during cyclic operating routine indicating whether the device is safe to be used or not.

Optionally, the device may further comprise a puff sensor that remain connected to the controller. The puffs sensor may be configured to detect each puff inhaled by the user during a smoking session. Further, to keep a track of number of puffs inhaled by the user, the controller may be configured to increment a puff counter with each puff. The controller may be configured to use this information to perform one or more functions of the device. For example, the controller may be able to decide the amount of consumable left in the smoking session based on total puffs available and number of puffs consumed.

The device may comprise an elongate body. An end of the elongate body may be configured for engagement with an aerosol-forming article. For example, the body may be configured for engagement with a heated tobacco (HT) consumable (or heat-not-burn (HNB) consumable). The terms "heated tobacco" and "heat-not-burn" are used interchangeably herein to describe a consumable that is of the type that is heated rather than combusted (or are used interchangeably to describe a device for use with such a consumable). The device may comprise a cavity that is configured for receipt of at least a portion of the consumable (i.e. for engagement with the consumable). The aerosol-forming article may be of the type that comprises an aerosol former (e.g. carried by an aerosol-forming substrate).

The device may comprise a heater for heating the aerosol-forming article. The heater may comprise a heating element, which may be in the form of a rod that extends from the body of the device. The heating element may extend from the end of the body that is configured for engagement with the aerosol-forming article.

The heater (and thus the heating element) may be rigidly mounted to the body. The heating element may be elongate so as to define a longitudinal axis and may, for example, have a transverse profile (i.e. transverse to a longitudinal axis of the heating element) that is substantially circular (i.e. the heating element may be generally cylindrical). Alternatively, the heating element may have a transverse profile that is rectangular (i.e. the heater may be a "blade heater"). The heating element may alternatively be in the shape of a tube (i.e. the heater may be a "tube heater"). The heating element may take other forms (e.g. the heating element may have an elliptical transverse profile). The shape and/or size (e.g. diameter) of the transverse profile of the heating element may be generally consistent for the entire length (or substantially the entire length) of the heating element.

The heating element may be between 15 mm and 25 mm long, e.g. between 18 mm and 20 mm long, e.g. around 19 mm long. The heating element may have a diameter of between 1.5 mm and 2.5 mm, e.g. a diameter between 2 mm and 2.3 mm, e.g. a diameter of around 2.15 mm.

The heating element may be formed of ceramic. The heating element may comprise a core (e.g. a ceramic core) comprising Al2O3. The core of the heating element may have a diameter of 1.8 mm to 2.1 mm, e.g. between 1.9 mm and 2 mm. The heating element may comprise an outer layer (e.g. an outer ceramic layer) comprising Al2O3. The thickness of the outer layer may be between 160 µm and 220 µm, e.g. between 170 µm and 190 µm, e.g. around 180 µm. The heating element may comprise a heating track, which may extend longitudinally along the heating element. The heating track may be sandwiched between the outer layer and the core of the heating element. The heating track may comprise tungsten and/or rhenium. The heating track may have a thickness of around 20 µm.

The heating element may be located in the cavity (of the device), and may extend (e.g. along a longitudinal axis) from an internal base of the cavity towards an opening of the cavity. The length of the heating element (i.e. along the longitudinal axis of the heater) may be less than the depth of the cavity. Hence, the heating element may extend for only a portion of the length of the cavity. That is, the heating element may not extend through (or beyond) the opening of the cavity.

The heating element may be configured for insertion into an aerosol-forming article (e.g. a HT consumable) when an aerosol-forming article is received in the cavity. In that respect, a distal end (i.e. distal from a base of the heating element where it is mounted to the device) of the heating element may comprise a tapered portion, which may facilitate insertion of the heating element into the aerosol-forming article. The heating element may fully penetrate an aerosol-forming article when the aerosol-forming article is received in the cavity. That is, the entire length, or substantially the entire length, of the heating element may be received in the aerosol-forming article.

The heating element may have a length that is less than, or substantially the same as, an axial length of an aerosol-forming substrate forming part of an aerosol-forming article (e.g. a HT consumable). Thus, when such an aerosol-forming article is engaged with the device, the heating element may only penetrate the aerosol-forming substrate, rather than other components of the aerosol-forming article. The heating element may penetrate the aerosol-forming substrate for substantially the entire axial length of the aerosol forming-substrate of the aerosol-forming article. Thus, heat may be transferred from (e.g. an outer circumferential surface of) the heating element to the surrounding aerosol-forming substrate, when penetrated by the heating element. That is, heat may be transferred radially outwardly (in the case of a cylindrical heating element) or e.g. radially inwardly (in the case of a tube heater).

Where the heater is a tube heater, the heating element of the tube heater may surround at least a portion of the cavity. When the portion of the aerosol-forming article is received in the cavity, the heating element may surround a portion of the aerosol-forming article (i.e. so as to heat that portion of the aerosol-forming article). In particular, the heating element may surround an aerosol forming substrate of the aerosol-forming article. That is, when an aerosol-forming article is engaged with the device, the aerosol forming substrate of the aerosol-forming article may be located adjacent an inner surface of the (tubular) heating element. When the heating element is activated, heat may be transferred radially inwardly from the inner surface of the heating element to heat the aerosol forming substrate.

The cavity may comprise a (e.g. circumferential) wall (or walls) and the (tubular) heating element may extend around at least a portion of the wall(s). In this way, the wall may be located between the inner surface of the heating element and an outer surface of the aerosol-forming article. The wall (or walls) of the cavity may be formed from a thermally conductive material (e.g. a metal) to allow heat conduction from the heating element to the aerosol-forming article. Thus, heat may be conducted from the heating element, through the cavity wall (or walls), to the aerosol-forming substrate of an aerosol-forming article received in the cavity.

In some embodiments the device may comprise a cap disposed at the end of the body that is configured for engagement with an aerosol-forming article. Where the device comprises a heater having a heating element, the cap may at least partially enclose the heating element. The cap may be moveable between an open position in which access is provided to the heating element, and a closed position in which the cap at least partially encloses the heating element. The cap may be slideably engaged with the body of the device, and may be slideable between the open and closed positions.

The cap may define at least a portion of the cavity of the device. That is, the cavity may be fully defined by the cap, or each of the cap and body may define a portion of the cavity. Where the cap fully defines the cavity, the cap may comprise an aperture for receipt of the heating element into the cavity (when the cap is in the closed position). The cap may comprise an opening to the cavity. The opening may be configured for receipt of at least a portion of an aerosol-forming article. That is, an aerosol-forming article may be inserted through the opening and into the cavity (so as to be engaged with the device).

The cap may be configured such that when an aerosol-forming article is engaged with the device (e.g. received in the cavity), only a portion of the aerosol-forming article is received in the cavity. That is, a portion of the aerosol-forming article (not received in the cavity) may protrude from (i.e. extend beyond) the opening. This (protruding) portion of the aerosol-forming article may be a terminal (e.g. mouth) end of the aerosol-forming article, which may be received in a user's mouth for the purpose of inhaling aerosol formed by the device.

The device may comprise a power source or may be connectable to a power source (e.g. a power source separate to the device). The power source may be electrically connectable to the heater. In that respect, altering (e.g. toggling) the electrical connection of the power source to the heater may affect a state of the heater. For example, toggling the electrical connection of the power source to the heater may toggle the heater between an on state and an off state. The power source may be a power store. For example, the power source may be a battery or rechargeable battery (e.g. a lithium ion battery).

The device may comprise an input connection (e.g. a USB port, Micro USB port, USB-C port, etc.). The input connection may be configured for connection to an external source of electrical power, such as a mains electrical supply outlet. The input connection may, in some cases, be used as a substitute for an internal power source (e.g. battery or rechargeable battery). That is, the input connection may be electrically connectable to the heater (for providing power to the heater). Hence, in some forms, the input connection may form at least part of the power source of the device.

Where the power source comprises a rechargeable power source (such as a rechargeable battery), the input connection may be used to charge and recharge the power source.

The device may comprise a user interface (Ul). In some embodiments the UI may include input means to receive operative commands from the user. The input means of the UI may allow the user to control at least one aspect of the operation of the device. In some embodiments the input means may comprise a power button to switch the device between an on state and an off state.

In some embodiments the UI may additionally or alternatively comprise output means to convey information to the user. In some embodiments, the output means may be configured to indicate the status of the device to the user. In some embodiments the output means may comprise a light to indicate a condition of the device (and/or the aerosol-forming article) to the user. The condition of the device (and/or aerosol-forming article) indicated to the user may comprise a condition indicative of the operation of the heater. For example, the condition may comprise whether the heater is in an off state or an on state. In some embodiments, the UI unit may comprise at least one of a button, a display, a touchscreen, a switch, a light, and the like. For example, the output means may comprise one or more (e.g. two, three, four, etc.) light-emitting diodes ("LEDs") that may be located on the body of the device.

The device may further comprise a puff sensor (e.g. airflow sensor), which form part of the input means of the Ul. The puff sensor may be configured to detect a user drawing on an end (i.e. a terminal (mouth) end) of the aerosol-forming article. The puff sensor may, for example, be a pressure sensor or a microphone. The puff sensor may be configured to produce a signal indicative of a puff state. The signal may be indicative of the user drawing (an aerosol from the aerosol-forming article) such that it is e.g. in the form of a binary signal. Alternatively or additionally, the signal may be indicative of a characteristic of the draw (e.g. a flow rate of the draw, length of time of the draw, etc). In some embodiment, the puff sensor may be configured to keep a track of each puff inhaled during a session. In an example, the puff sensor may be configured to increment a puff counter with each puff inhaled, to keep a count of each puff inhaled, during a session. By doing so the puff sensor may help in determining the amount of consumable available for consumption in said session.

The device may comprise a controller or may be connectable to a controller that may be configured to control at least one function of the device. The controller may comprise a microcontroller that may e.g. be mounted on a printed circuit board (PCB). The controller may also comprise a memory, e.g. nonvolatile memory. The memory may include instructions, which, when implemented, may cause the controller to perform certain tasks or steps of a method. Where the device comprises an input connection, the controller may be connected to the input connection.

The controller may be configured to control the operation of the heater (and e.g. the heating element). Thus, the controller may be configured to control vaporisation of an aerosol forming part of an aerosol-forming article engaged with the device. Further, the controller may be configured to run a cyclic operating routine in the device with a period between 10ms to 500ms. The controller may be configured to perform a number of functions during the cyclic operating routine to ensure safety of the device. In addition, the controller may be configured to control the voltage applied by power source to the heater. For example, the controller may be configured to toggle between applying a full output voltage (of the power source) to the heater and applying no voltage to the heater. Alternatively or additionally, the control unit may implement a more complex heater control protocol.

The device may further comprise a voltage regulator to regulate the output voltage supplied by the power source to form a regulated voltage. The regulated voltage may subsequently be applied to the heater.

In some embodiments, where the device comprises a Ul, the controller may be operatively connected to one or more components of the Ul. The controller may be configured to receive command signals from an input means of the Ul. The controller may be configured to control the heater in response to the command signals. For example, the controller may be configured to receive "on" and "off" command signals from the UI and, in response, may control the heater so as to be in a corresponding on or off state.

The controller may be configured to send output signals to a component of the Ul. The UI may be configured to convey information to a user, via an output means, in response to such output signals (received from the controller). For example, where the device comprises one or more LEDs, the LEDs may be operatively connected to the controller. Hence, the controller may configured to control the illumination of the LEDs (e.g. in response to an output signal). For example, the controller may be configured to control the illumination of the LEDs according to (e.g. an on or off) state of the heater. Further in an example, the UI may include other output means that may be configured to provide one of audio, visual and haptic feedback, to the user, indicating the status of the device.

Where the device comprises a sensor (e.g. a puff/airflow sensor), the controller may be operatively connected to the sensor. The controller may be configured to receive a signal from the sensor (e.g. indicative of a condition of the device and/or engaged aerosol-forming article). The controller may be configured to control the heater, or an aspect of the output means, based on the signal from the sensor. In one example, the puff sensor may be configured to count the number of puffs inhaled during a session and pass this information to the controller. The controller may then utilize this information to determine for example, the amount of consumable remaining in the session.

The device may comprise a wireless interface configured to communicate wirelessly (e.g. via Bluetooth (e.g. a Bluetooth low-energy connection) or WiFi) with an external device. Similarly, the input connection may be configured for wired connection to an external device so as to provide communication between the device and the external device.

The external device may be a mobile device. For example, the external device may be a smart phone, tablet, smart watch, or smart car. An application (e.g. app) may be installed on the external device (e.g. mobile device). The application may facilitate communication between the device and the external device via the wired or wireless connection.

The wireless or wired interface may be configured to transfer signals between the external device and the controller of the device. In this respect, the controller may control an aspect of the device in response to a signal received from an external device. Alternatively or additionally, an external device may respond to a signal received from the device (e.g. from the controller of the device).

In a second aspect, there is provided a system (e.g. a smoking substitute system) comprising a device according to the first aspect and an aerosol-forming article. The aerosol-forming article may comprise an aerosol-forming substrate at an upstream end of the aerosol-forming article. The article may be in the form of a smoking substitute article, e.g. heated tobacco (HT) consumable (also known as a heat-not-burn (HNB) consumable).

As used herein, the terms "upstream" and "downstream" are intended to refer to the flow direction of the vapour/aerosol i.e. with the downstream end of the article/consumable being the mouth end or outlet where the aerosol exits the consumable for inhalation by the user. The upstream end of the article/consumable is the opposing end to the downstream end.

The aerosol-forming substrate is capable of being heated to release at least one volatile compound that can form an aerosol. The aerosol-forming substrate may be located at the upstream end of the article/consumable.

In order to generate an aerosol, the aerosol-forming substrate comprises at least one volatile compound that is intended to be vaporised/aerosolised and that may provide the user with a recreational and/or medicinal effect when inhaled. Suitable chemical and/or physiologically active volatile compounds include the group consisting of: nicotine, cocaine, caffeine, opiates and opoids, cathine and cathinone, kavalactones, mysticin, beta-carboline alkaloids, salvinorin A together with any combinations, functional equivalents to, and/or synthetic alternatives of the foregoing.

The aerosol-forming substrate may comprise plant material. The plant material may comprise least one plant material selected from the list including *Amaranthus dubius, Arctostaphylos uva-ursi* (Bearberry), *Argemone mexicana, Amica, Artemisia vulgaris,* Yellow Tees, *Galea zacatechichi, Canavalia maritima* (Baybean), *Cecropia mexicana* (Guamura), *Cestrum noctumum, Cynoglossum virginianum* (wild comfrey), *Cytisus scoparius, Damiana, Entada rheedii, Eschscholzia califomica* (California Poppy), *Fittonia albivenis, Hippobroma longiflora, Humulus japonica* (Japanese Hops), *Humulus lupulus* (Hops), *Lactuca virosa* (Lettuce Opium), *Laggera alata, Leonotis leonurus, Leonurus cardiaca* (Motherwort), *Leonurus sibiricus* (Honeyweed), *Lobelia cardinalis, Lobelia inflata* (Indian-tobacco), *Lobelia siphilitica, Nepeta cataria* (Catnip), *Nicotiana species* (Tobacco), *Nymphaea alba* (White Lily), *Nymphaea caerulea* (Blue Lily), Opium poppy, *Passiflora incamata* (Passionflower), *Pedicularis densiflora* (Indian Warrior), *Pedicularis groenlandica* (Elephant's Head), *Salvia divinorum, Salvia dorrii* (Tobacco Sage), Salvia species (Sage), *Scutellaria galericulata, Scutellaria lateriflora, Scutellaria nana, Scutellaria* species (Skullcap), *Sida acuta* (Wireweed), *Sida rhombifolia, Silene capensis, Syzygium aromaticum* (Clove), *Tagetes lucida* (Mexican Tarragon), *Tarchonanthus camphoratus, Tumera diffusa* (Damiana), *Verbascum* (Mullein), *Zamia latifolia* (Maconha Brava) together with any combinations, functional equivalents to, and/or synthetic alternatives of the foregoing.

The plant material may be tobacco. Any type of tobacco may be used. This includes, but is not limited to, flue-cured tobacco, burley tobacco, Maryland Tobacco, dark-air cured tobacco, oriental tobacco, dark-fired tobacco, perique tobacco and rustica tobacco. This also includes blends of the above mentioned tobaccos.

The tobacco may comprise one or more of leaf tobacco, stem tobacco, tobacco powder, tobacco dust, tobacco derivatives, expanded tobacco, homogenised tobacco, shredded tobacco, extruded tobacco, cut rag tobacco and/or reconstituted tobacco (e.g. slurry recon or paper recon).

The aerosol-forming substrate may comprise a gathered sheet of homogenised (e.g. paper/slurry recon) tobacco or gathered shreds/strips formed from such a sheet.

The aerosol-forming substrate may comprise one or more additives selected from humectants, flavourants, fillers, aqueous/non-aqueous solvents and binders.

The flavourant may be provided in solid or liquid form. It may include menthol, liquorice, chocolate, fruit flavour (including e.g. citrus, cherry etc.), vanilla, spice (e.g. ginger, cinnamon) and tobacco flavour. The flavourant may be evenly dispersed throughout the aerosol-forming substrate or may be provided in isolated locations and/or varying concentrations throughout the aerosol-forming substrate.

The aerosol-forming substrate may be formed in a substantially cylindrical shape such that the article/consumable resembles a conventional cigarette. It may have a diameter of between 5 and 10mm e.g. between 6 and 9mm or 6 and 8mm e.g. around 7 mm. It may have an axial length of between 10 and 15mm e.g. between 11 and 14mm such as around 12 or 13mm.

The article/consumable may comprise at least one filter element. There may be a terminal filter element at the downstream/mouth end of the article/consumable.

The or at least one of the filter element(s) (e.g. the terminal filter element) may be comprised of cellulose acetate or polypropylene tow. The at least one filter element (e.g. the terminal filter element) may be comprised of activated charcoal. The at least one filter element (e.g. the terminal element) may be comprised of paper. The or each filter element may be at least partly (e.g. entirely) circumscribed with a plug wrap e.g. a paper plug wrap.

The terminal filter element (at the downstream end of the article/consumable) may be joined to the upstream elements forming the article/consumable by a circumscribing tipping layer e.g. a tipping paper layer. The tipping paper may have an axial length longer than the axial length of the terminal filter element such that the tipping paper completely circumscribes the terminal filter element plus the wrapping layer surrounding any adjacent upstream element.

In some embodiments, the article/consumable may comprise an aerosol-cooling element which is adapted to cool the aerosol generated from the aerosol-forming substrate (by heat exchange) before being inhaled by the user.

The article/consumable may comprise a spacer element that defines a space or cavity between the aerosol-forming substrate and the downstream end of the consumable. The spacer element may comprise a cardboard tube. The spacer element may be circumscribed by the (paper) wrapping layer.

According to a third aspect of the present invention, there is provided a method of using the system according to the second aspect, the method comprising inserting the aerosol-forming article into the device; and heating the article using the heater of the device.

In some embodiments the method may comprise inserting the article into a cavity within a body of the device and penetrating the article with the heating element of the device upon insertion of the article.

The invention includes the combination of the aspects and preferred features described except where such a combination is clearly impermissible or expressly avoided.

The skilled person will appreciate that except where mutually exclusive, a feature or parameter described in relation to any one of the above aspects may be applied to any other aspect. Furthermore, except where mutually exclusive, any feature or parameter described herein may be applied to any aspect and/or combined with any other feature or parameter described herein.

### SUMMARY OF THE FIGURES

So that the invention may be understood, and so that further aspects and features thereof may be appreciated, embodiments illustrating the principles of the invention will now be discussed in further detail with reference to the accompanying figures, in which:
Figure 1A is a schematic of a smoking substitute system;
Figure 1B is a schematic of a variation of the smoking substitute system of Figure 1A;
Figure 2A is a front view of a first embodiment of a smoking substitute system with the consumable engaged with the device;
Figure 2B is a front view of the first embodiment of the smoking substitute system with the consumable disengaged from the device;
Figure 2C is a section view of the consumable of the first embodiment of the smoking substitute system;
Figure 2D is a detailed view of an end of the device of the first embodiment of the smoking substitute system;
Figure 2E is a section view of the first embodiment of the substitute smoking system.

### DETAILED DESCRIPTION OF THE INVENTION

Aspects and embodiments of the present invention will now be discussed with reference to the accompanying figures. Further aspects and embodiments will be apparent to those skilled in the art. All documents mentioned in this text are incorporated herein by reference.

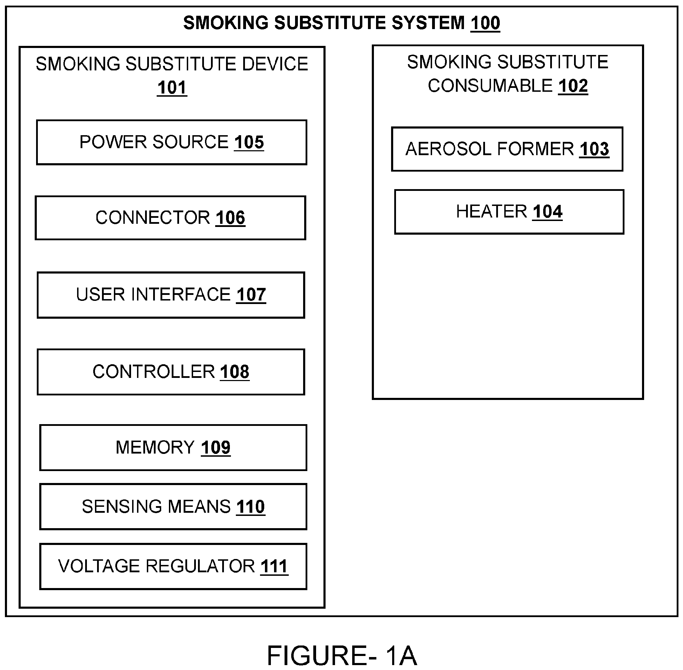

Figure 1A is a schematic providing a general overview of a smoking substitute system 100. The system 100 includes a substitute smoking device 101 and an aerosol-forming article in the form of a consumable 102, which comprises an aerosol former 103. The system is configured to vaporise the aerosol former by heating the aerosol former 103 (so as to form a vapour/aerosol for inhalation by a user).

In the illustrated system, the heater 104 forms part of the consumable 102 and is configured to heat the aerosol former 103. Heat from the heater 104 vaporises the aerosol former 103 to produce a vapour. The vapour subsequently condenses to form an aerosol, which is ultimately inhaled by the user.

The system 100 further comprises a power source 105 that forms part of the device 101. In other embodiments the power source 105 may be external to (but connectable to) the device 101. The power source 105 is electrically connectable to the heater 104 such that the power source 105 is able to supply power to the heater 104 (i.e. for the purpose of heating the aerosol former 103). Thus, control of the electrical connection of the power source 105 to the heater 104 provides control of the state of the heater 104. The power source 105 may be a power store, for example a battery or rechargeable battery (e.g. a lithium ion battery).

The system 100 further comprises an I/O module comprising a connector 106 (e.g. in the form of a USB port, Micro USB port, USB-C port, etc.). The connector 106 is configured for connection to an external source of electrical power, e.g. a mains electrical supply outlet. The connector 106 may be used in substitution for the power source 105. That is the connector 106 may be electrically connectable to the heater 104 so as to supply electricity to the heater 104. In such embodiments, the device may not include a power source, and the power source of the system may instead comprise the connector 106 and an external source of electrical power (to which the connector 106 provides electrical connection).

In some embodiments, the connector 106 may be used to charge and recharge the power source 105 where the power source 104 includes a rechargeable battery.

The system 100 also comprises a user interface (Ul) 107. Although not shown, the Ul 107 may include input means to receive commands from a user. The input means of the UI 107 allows the user to control at least one aspect of the operation of the system 100. The input means may, for example, be in the form of a button, touchscreen, switch, microphone, etc.

The Ul 107 also comprises output means to convey information to the user. The output means may, for example, comprise lights (e.g. LEDs), a display screen, speaker, vibration generator, etc.

The system 100 further comprises a controller 108 and a memory 109 operatively coupled to the controller 108.that is configured to control at least one function of the device 101. In the illustrated embodiment, the controller 108 is a component of the device 101, but in other embodiments may be separate from (but connectable to) the device 101. The controller 108 is configured to perform at least one function for the device during a cyclic operating routine. The controller 108 is further configured to control the operation of the heater 104 and, for example, may be configured to control the voltage applied from the power source 105 to the heater 104. The controller 108 may be configured to toggle the supply of power to the heater 105 between an on state, in which the full output voltage of the power source 105 is applied to the heater 104, and an off state, in which the no voltage is applied to the heater 104.

Although not shown, the system 100 may also comprise a voltage regulator to regulate the output voltage from the power source 105 to form a regulated voltage. The regulated voltage may then be applied to the heater 104.

In addition to being connected to the heater 104, the controller 108 is operatively connected to the UI 107. Thus, the controller 108 may receive an input signal from the input means of the UI 107. Similarly, the controller 108 may transmit output signals to the UI 107. In response, the output means of the UI 107 may convey information, based on the output signals, to a user.

Further, the system 100 may comprise a sensing means 110 coupled with the controller 108 within the device 101. The sensing means 110 may include a puff sensor mounted inside the device 101 and configured to detect a user puff. The controller may count the number of number of puffs during a consumable cycle. The sensor 110 may include a heater temperature sensor configured to measure a heater temperature of the heater. The sensing means 110 may include an ambient temperature sensor configured to measure ambient temperature. The sensing means 110 may include a cap movement sensor configured to detect one of removal and lift of a cap from the device 101. The cap movement sensor may be a hall effect sensor.

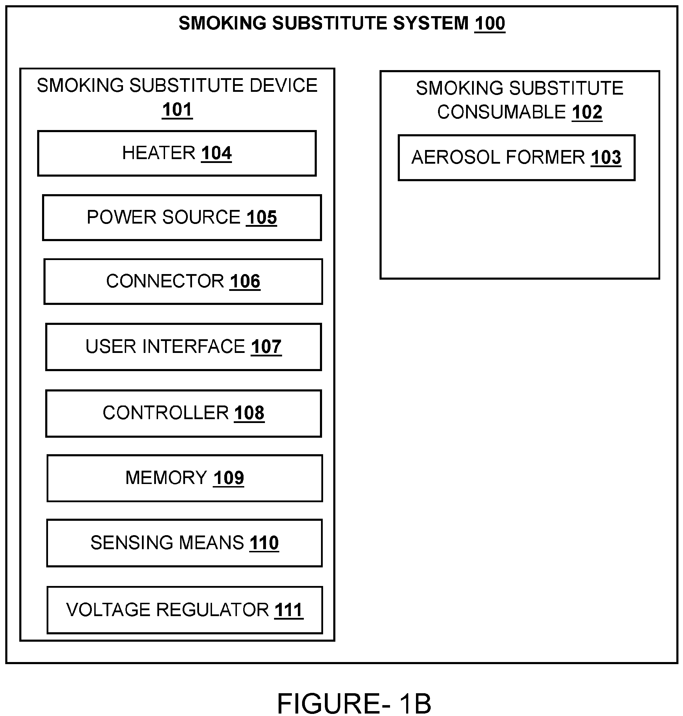

Figure 1B is a schematic showing a variation of the system 100 of Figure 1A. In the system 100' of Figure 1B, the heater 104 forms part of the consumable 102, rather than the device 101. In this variation, the heater 104 is electrically connectable to the power source 105, for example, when the consumable 102 is engaged with the device 101.

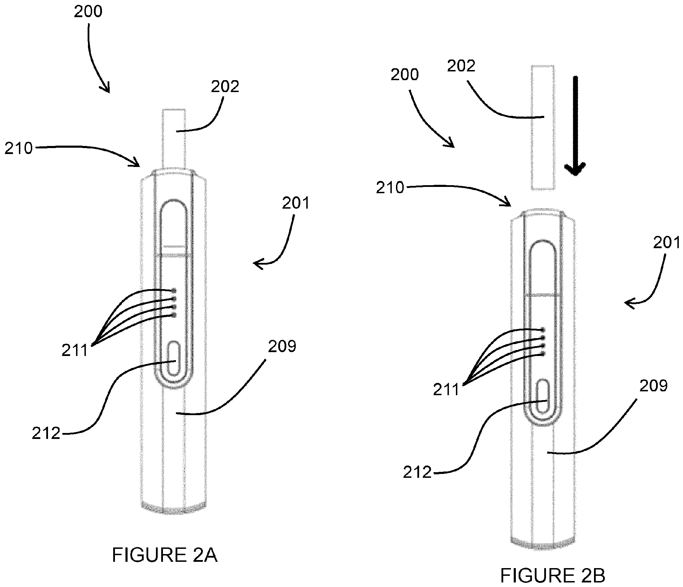

Figures 2A and 2B illustrate a heated-tobacco (HT) smoking substitute system 200. The system 200 is an example of the systems 100, 100' described in relation to Figures 1A or 1B. System 200 includes an HT device 201 and an HT consumable 202. The description of Figures 1A and 1B above is applicable to the system 200 of Figures 2A and 2B, and will thus not be repeated.

The device 201 and the consumable 202 are configured such that the consumable 202 can be engaged with the device 201. Figure 2A shows the device 201 and the consumable 202 in an engaged state, whilst Figure 2B shows the device 201 and the consumable 202 in a disengaged state.

The device 201 comprises a body 209 and cap 210. In use the cap 209 is engaged at an end of the body 209. Although not apparent from the figures, the cap 210 is moveable relative to the body 209. In particular, the cap 210 is slideable and can slide along a longitudinal axis of the body 209.

The device 201 comprises an output means (forming part of the UI of the device 201) in the form of a plurality of light-emitting diodes (LEDs) 211 arranged linearly along the longitudinal axis of the device 201 and on an outer surface of the body 209 of the device 201. A button 212 is also arranged on an outer surface of the body 209 of the device 201 and is axially spaced (i.e. along the longitudinal axis) from the plurality of LEDs 211.

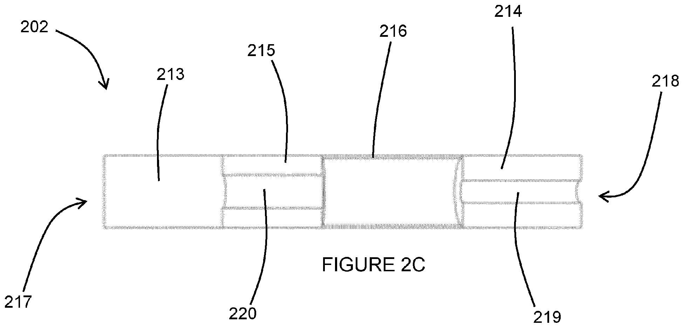

Figure 2C show a detailed section view of the consumable of 202 of the system 200. The consumable 202 generally resembles a cigarette. In that respect, the consumable 202 has a generally cylindrical form with a diameter of 7 mm and an axial length of 70 mm. The consumable 202 comprises an aerosol forming substrate 213, a terminal filter element 214, an upstream filter element 215 and a spacer element 216. In other embodiments, the consumable may further comprise a cooling element. A cooling element may exchange heat with vapour that is formed by the aerosol-forming substrate 213 in order to cool the vapour so as to facilitate condensation of the vapour.

The aerosol-forming substrate 213 is substantially cylindrical and is located at an upstream end 217 of the consumable 202, and comprises the aerosol former of the system 200. In that respect, the aerosol forming substrate 213 is configured to be heated by the device 201 to release a vapour. The released vapour is subsequently entrained in an airflow flowing through the aerosol-forming substrate 213. The airflow is produced by the action of the user drawing on a downstream 218 (i.e. terminal or mouth end) of the consumable 202.

In the present embodiment, the aerosol forming substrate 213 comprises tobacco material that may, for example, include any suitable parts of the tobacco plant (e.g. leaves, stems, roots, bark, seeds and flowers). The tobacco may comprise one or more of leaf tobacco, stem tobacco, tobacco powder, tobacco dust, tobacco derivatives, expanded tobacco, homogenised tobacco, shredded tobacco, extruded tobacco, cut rag tobacco and/or reconstituted tobacco (e.g. slurry recon or paper recon). For example, the aerosol-forming substrate 213 may comprise a gathered sheet of homogenised (e.g. paper/slurry recon) tobacco or gathered shreds/strips formed from such a sheet.

In order to generate an aerosol, the aerosol forming substrate 213 comprises at least one volatile compound that is intended to be vaporised/aerosolised and that may provide the user with a recreational and/or medicinal effect when inhaled. The aerosol-forming substrate 213 may further comprise one or more additives. For example, such additives may be in the form of humectants (e.g. propylene glycol and/or vegetable glycerine), flavourants, fillers, aqueous/non-aqueous solvents and/or binders.

The terminal filter element 214 is also substantially cylindrical, and is located downstream of the aerosol forming substrate 213 at the downstream end 218 of the consumable 202. The terminal filter element 214 is in the form of a hollow bore filter element having a bore 219 (e.g. for airflow) formed therethrough. The diameter of the bore 219 is 2 mm. The terminal filter element 214 is formed of a porous (e.g. monoacetate) filter material. As set forth above, the downstream end 218 of the consumable 202 (i.e. where the terminal filter 214 is located) forms a mouthpiece portion of the consumable 202 upon which the user draws. Airflow is drawn from the upstream end 217, thorough the components of the consumable 202, and out of the downstream end 218. The airflow is driven by the user drawing on the downstream end 218 (i.e. the mouthpiece portion) of the consumable 202.

The upstream filter element 215 is located axially adjacent to the aerosol-forming substrate 213, between the aerosol-forming substrate 213 and the terminal filter element 214. Like the terminal filter 214, the upstream filter element 215 is in the form of a hollow bore filter element, such that it has a bore 220 extending axially therethrough. In this way, the upstream filter 215 may act as an airflow restrictor. The upstream filter element 215 is formed of a porous (e.g. monoacetate) filter material. The bore 220 of the upstream filter element 214 has a larger diameter (3 mm) than the terminal filter element 214.

The spacer 216 is in the form of a cardboard tube, which defines a cavity or chamber between the upstream filter element 215 and the terminal filter element 214. The spacer 216 acts to allow both cooling and mixing of the vapour/aerosol from the aerosol-forming substrate 213. The spacer has an external diameter of 7 mm and an axial length of 14mm.

Although not apparent from the figure, the aerosol-forming substrate 213, upstream filter 215 and spacer 216 are circumscribed by a paper wrapping layer. The terminal filter 214 is circumscribed by a tipping layer that also circumscribes a portion of the paper wrapping layer (so as to connect the terminal filter 214 to the remaining components of the consumable 202). The upstream filter 215 and terminal filter 214 are circumscribed by further wrapping layers in the form of plug wraps.

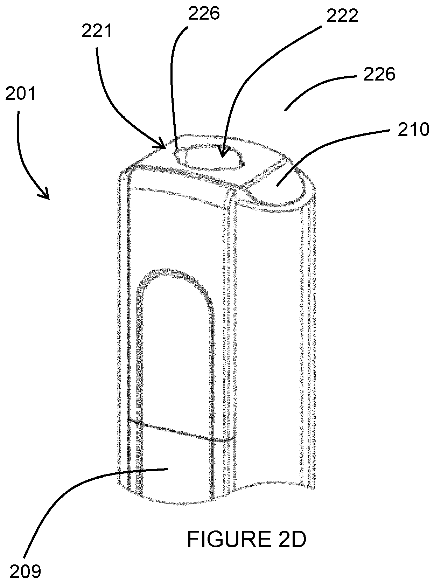

Returning now to the device 201, Figure 2D illustrates a detailed view of the end of the device 201 that is configured to engage with the consumable 202. The cap 210 of the device 201 includes an opening 221 to an internal cavity 222 (more apparent from Figure 2D) defined by the cap 210. The opening 221 and the cavity 222 are formed so as to receive at least a portion of the consumable 202. During engagement of the consumable 202 with the device 201, a portion of the consumable 202 is received through the opening 221 and into the cavity 222. After engagement (see Figure 2B), the downstream end 218 of the consumable 202 protrudes from the opening 221 and thus also protrudes from the device 201. The opening 221 includes laterally disposed notches 226. When a consumable 202 is received in the opening 221, these notches 226 remain open and could, for example, be used for retaining a cover in order to cover the end of the device 201.

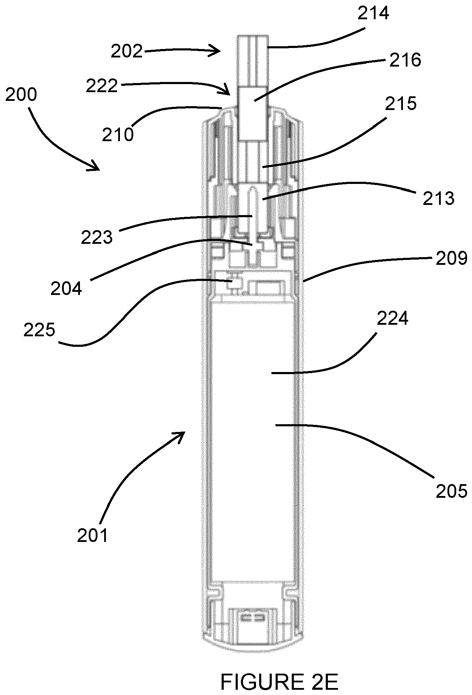

Figure 2E shows a cross section through a central longitudinal plane through the device 201. The device 201 is shown with the consumable 202 engaged therewith.

The device 201 comprises a heater 204 comprising heating element 223. The heater 204 forms part of the body 209 of the device 201 and is rigidly mounted to the body 209. In the illustrated embodiment, the heater 204 is a rod heater with a heating element 223 having a circular transverse profile. In other embodiments the heater may be in the form of a blade heater (e.g. heating element with a rectangular transverse profile) or a tube heater (e.g. heating element with a tubular form).

The heating element 223 of the heater 204 projects from an internal base of the cavity 222 along a longitudinal axis towards the opening 221. As is apparent from the figure, the length (i.e. along the longitudinal axis) of the heating element is less than a depth of the cavity 222. In this way, the heating element 223 does not protrude from or extend beyond the opening 221.

When the consumable 202 is received in the cavity 222 (as is shown in Figure 2E), the heating element 223 penetrates the aerosol-forming substrate 213 of the consumable 202. In particular, the heating element 223 extends for nearly the entire axial length of the aerosol-forming substrate 213 when inserted therein. Thus, when the heater 204 is activated, heat is transferred radially from an outer circumferential surface the heating element 223 to the aerosol-forming substrate 213.

The device 201 further comprises an electronics cavity 224. A power source, in the form of a rechargeable battery 205 (a lithium ion battery), is located in electronics cavity 224.

The device 201 includes a connector (i.e. forming part of an IO module of the device 201) in the form of a USB port 206. The connector may alternatively be, for example, a micro-USB port or a USB-C port for examples. The USB port 206 may be used to recharge the rechargeable battery 205.

The device 201 includes a controller (not shown) located in the electronics cavity 224. The controller comprises a microcontroller mounted on a printed circuit board (PCB). The USB port 206 is also connected to the controller 208 (i.e. connected to the PCB and microcontroller).

The controller 208 is configured to control at least one function of the device 202. For example, the controller 208 is configured to control the operation of the heater 204. Such control of the operation of the heater 204 may be accomplished by the controller toggling the electrical connection of the rechargeable battery 205 to the heater 204. For example, the controller 208 is configured to control the heater 204 in response to a user depressing the button 212. Depressing the button 212 may cause the controller to allow a voltage (from the rechargeable battery 205) to be applied to the heater 204 (so as to cause the heating element 223 to be heated).

The controller 208 is configured with a cyclic operating routine having a period between an upper period boundary and lower period boundary.

The upper period boundary may be selected from any one of 500 milliseconds ("ms"), 450 ms, 400 ms, 350 ms, 300 ms, 250 ms, 200 ms, 150 ms, 100 ms, 50 ms.

The lower period boundary may be selected from any one of 10 ms, 20 ms, 30 ms, 40 ms, 50 ms. Where consistent with the selected upper period boundary, the lower period boundary may alternatively be selected from 100 ms, 150 ms, 200 ms, 250 ms, 300 ms, 350 ms, 400 ms, 450 ms.

By way of examples, the period may be between 150 ms and 400 ms, or; the period may be between 20 ms and 50 ms. In some embodiments, the period is between 10 ms and 500 ms.

The cyclic operating routine repeats once every period. In one example, the controller 208 may be configured to run a single cycle of cyclic operating routine or multiple cycles of the cyclic operating routine. During each cycle of the cyclic operating routine, the controller 208 is configured to perform at least one function, among a number of functions, for the device 201. By performing at least one function during the cyclic operating routine, the controller 208 may ensure enhanced safety of the device 201. In particular, the relatively long period of an operating routine of the present invention may provide a balance between user experience, speed of operation, and computational stability of the controller 208.

A number of potential functions performed during the cyclic operating routine are described below.

In some embodiments, the functions performed by the controller 208 during the cyclic operating routine may comprise measuring the temperature of the heater 204 during the cyclic operating routine. The controller 208 may be configured to measure the temperature of the heater 204 once each period of the cyclic operating routine. Further, to measure the temperature of the heater 204, a heater temperature sensor 110 may be used by the controller 208. Upon receiving the measured temperature for the heater 204, the controller 208 may compare it to a predetermined threshold temperature. The value of threshold temperature may be pre-stored in the memory of the device 201. In response to said comparison, the controller 208 may be configured to control the operation of the heater 204. In particular, the power supplied to the heater 204 may be controlled. For example, if the temperature of the heater 204 is above the threshold, the power supplied to the heater 204 may be reduced or prevented entirely. For this, the controller 208 may be configured to send a signal to power source 106, indicating that the power supply to the heater 204 must be reduced. Using this function, the heater 204 may be controlled to be substantially maintained at an operating temperature.

In some embodiments, the controller 208 may be configured to regulate the voltage supply to the heater 204. The controller 208 may work in conjunction with the voltage regulator 111 to regulate the voltage supply to the heater 204. In an embodiment, if the controller 208 detects that the voltage supply to the heater 204 needs to be regulated, i.e. increased or decreased, the controller 208 sends a corresponding signal to the voltage regulator 111. In some embodiments, the controller 208 only updates an on or off state of the supply of power to the heater once per operating cycle.

In some embodiments, the functions performed by the controller 208 during each period of the cyclic operating routine may comprise measuring an output voltage of a power supply of the device. The power supplied to the heater 204 may be controlled by the controller based on the measured output voltage of the power supply. In particular, the duty cycle of pulse width modulated power supply to the heater may be increased as the output voltage of the power supply decreases to maintain a constant delivery of power to the heater.

In some embodiments, the functions performed by the controller 208 during each period of the cyclic operating routine may comprise measuring an ambient temperature for the device using the ambient temperature sensor 110. In such an embodiment, the ambient temperature sensor 110 may be a temperature sensor placed inside the device 201 configured to measure the ambient temperature. Based on the ambient temperature, the controller 208 may be configured to control various operations of the device 201. In one example, the controller 208 may be configured to disable the heater 204 in response to detecting an ambient temperature above a high ambient temperature threshold (e.g. 55 degrees Celsius). In another example, the controller 208 may be configured to deactivate one of a plurality of operating modes (for example, disable a mode in which the heater 204 would be heated to a higher operating temperature).

In some embodiments, the functions performed by the controller 208 during each period of cyclic operating routine includes detecting the status of a puff sensor.. In such embodiments, the sensor 110 may be a puff sensor placed inside the cavity 222 of the device 201 configured to measure puffs taken by the user. The puff sensor 110 may further be configured to increment a puff counter (not shown) with each puff. Said puff counter helps the controller 208 in tracking the number of puffs inhaled and number of puffs remaining in the session. Based on said information the controller 208 may be configured to control one or more important operations of the device 201 and provide useful indication to the user. For example, based on the puff count, the controller 208 may be configured to determine the amount of consumable left in the session. The controller 208 may be configured to alert the user as to how much time is left before the consumable is exhausted.

In some embodiments, the functions performed by the controller 208 during each period of cyclic operating routine include detecting the position of the cap 210 within the device 201. Precisely, the controller 208 may be configured to detect the removal or lifting of the cap 210 from the device 201. For this, the controller 208 may be configured to use the sensor 210. In such embodiment, the sensor 110 may be a hall-effect sensor placed inside the cavity 222 of the device 201 configured to detect the change in pressure difference created, within the cavity 222, due to one of insertion and removal of the cap 210.

In some embodiments, the functions performed by the controller 208 during each period of cyclic operating routine include detecting a current state of the device. Current state may include for example, operational state, standby state, off state, error state. Precisely, the controller 208 may be configured to detect the removal or lifting of the cap 210 from the device 201. For this, the controller 208 may be configured to use the sensor 210. In such embodiment, the sensor 110 may be a hall-effect sensor placed inside the cavity 222 of the device 201 configured to detect the change in pressure difference created, within the cavity 222, due to one of insertion and removal of the cap 210.

The device 201 may further include an output means (not shown) connected to the controller 208. In one embodiment, the output means may form a part of the UI 107. The output means may include at least one plurality of LEDs, a haptic sensor, a microphone or any other similar means that may be configured to provide at least one of audio, visual and haptic feedback, indicating the status update of the device 201, to the user. In some embodiments, the functions performed by the controller 208 during each period of cyclic operating routine include updating the output status of the output means to indicate information to the user. In some embodiments, a charge level is determined once per operating cycle and the output means updated to reflect the charge level.

The device 201 may further include an input means (not shown) connected to the controller 208. In one embodiment, the input means may form a part of the UI 107. The input means may include a button. In some embodiments, the functions performed by the controller 208 during each period of cyclic operating routine include making a determination of whether the user has made an input command to the input means. During the cycle the controller 208 may react to a detected user input command if one is determined to have taken place. The user input command may include a predefined sequence of button presses. The predefined sequence of button presses may include a predefined frequency, duration or pattern of button presses.

The controller 208 is also configured to control the LEDs 211 in response to (e.g. a detected) a condition of the device 201 or the consumable 202. For example, the controller may control the LEDs to indicate whether the device 201 is in an on state or an off state (e.g. one or more of the LEDs may be illuminated by the controller when the device is in an on state).

The device 202 comprises a plurality of input means (i.e. in addition to the button 212) in the form of the puff sensor 225, audio sensor, motion sensor etc. Said other input means may be configured to receive user input in the corresponding manner. Further, similar to output means, the input means may also form a part of UI 107. As discussed, the puff sensor 225 is configured to detect a user drawing (i.e. inhaling) at the downstream end 218 of the consumable 202. The puff sensor 225 may, for example, be in the form of a pressure sensor, flowmeter or a microphone. The puff sensor 225 is operatively connected to the controller 208 in the electronics cavity 224, such that a signal from the puff sensor 225, indicative of a puff state (i.e. drawing or not drawing), forms an input to the controller 208 (and can thus be responded to by the controller 208). The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the words "have", "comprise", and "include", and variations such as "having", "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means, for example, +/- 10%.

The words "preferred" and "preferably" are used herein refer to embodiments of the invention that may provide certain benefits under some circumstances. It is to be appreciated, however, that other embodiments may also be preferred under the same or different circumstances. The recitation of one or more preferred embodiments therefore does not mean or imply that other embodiments are not useful, and is not intended to exclude other embodiments from the scope of the disclosure, or from the scope of the claims.

Features of embodiments of the invention are set out in the following paragraphs.

Clause 1. A heat not burn device including a controller having a cyclic operating routine, wherein the controller is configured to, during each cycle of the cyclic operating routine, perform at least one function of the device; and wherein a period of the cyclic operating routine is between 10 milliseconds and 500 milliseconds.

Clause 2. The device according to clause 1, wherein said period of the cyclic operating routine is between 150 milliseconds and 400 milliseconds.

Clause 3. A device according to clause 1 or clause 2, wherein at least one of the at least one function is performed exactly once during each cycle of the cyclic operating routine.

Clause 4. A device according to clause 3, wherein each of the at least one function is performed exactly once during each cycle of the cyclic operating routine.

Clause 5. The device according to any preceding clause, wherein the at least one function includes measuring a heater temperature of a heater of the device.

Clause 6. The device according to clause 5, wherein the at least one function includes comparing the heater temperature with a predetermined heater threshold.

Clause 7. The device according to clause 6, wherein the at least one function includes controlling a power supplied to the heater based on the comparison between the heater temperature and the predetermined heater threshold.

Clause 8. The device according to any preceding clause, further comprising a voltage detection means connected to the controller, and the at least one function including using the voltage detection means to measure an output voltage of a power supply of the device.

Clause 9. The device according to clause 8, wherein the at least one function includes controlling a power supplied to the heater based on the measured output voltage.

Clause 10. The device according to any preceding clause, further comprising an ambient temperature sensor connected to the controller and wherein the at least one function includes measuring an ambient temperature for the device.

Clause 11. The device according to clause 10, wherein the at least one function includes controlling a power supplied to the heater based on the measured ambient temperature.

Clause 12. The device according to any preceding clause, wherein the at least one function includes making a determination of the presence of a user input command to an input of a user interface of the device.

Clause 13. The device according to any preceding clause, wherein the at least one function includes updating an output of a user interface of the device.

Clause 14. The device according to any preceding clause, wherein the at least one function includes determining a puff status of a puff sensing means of the device.

Clause 15. The device according to any preceding clause, wherein the at least one function includes making a determination of a charging status of a power supply of the device.

Clause 16. The device according to any preceding clause, wherein the controller includes a microcontroller or a microprocessor.

## Claims

1. A heat not burn device (101, 201) including a controller (108, 208) having a cyclic operating routine, wherein the controller (108, 208) is configured to, during each cycle of the cyclic operating routine, perform at least one function of the device (101, 201); wherein a period of the cyclic operating routine is between 10 milliseconds and 500 milliseconds; and
wherein either:
i) the device further comprises voltage detection means connected to the controller, and the at least one function includes using the voltage detection means to measure an output voltage of a power supply of the device; or
ii) the device further comprises an ambient temperature sensor connected to the controller, and the at least one function includes measuring an ambient temperature for the device.

2. The device according to claim 1, wherein said period of the cyclic operating routine is between 150 milliseconds and 400 milliseconds.

3. A device according to claim 1 or claim 2, wherein at least one of the at least one function is performed exactly once during each cycle of the cyclic operating routine.

4. A device according to claim 3, wherein each of the at least one function is performed exactly once during each cycle of the cyclic operating routine.

5. The device according to any preceding claim, wherein the at least one function includes measuring a heater temperature of a heater of the device.

6. The device according to claim 5, wherein the at least one function includes comparing the heater temperature with a predetermined heater threshold.

7. The device according to claim 6, wherein the at least one function includes controlling a power supplied to the heater based on the comparison between the heater temperature and the predetermined heater threshold.

8. The device according to any preceding claim, comprising the voltage detection means connected to the controller, and the at least one function including using the voltage detection means to measure an output voltage of a power supply of the device.

9. The device according to claim 8, wherein the at least one function includes controlling a power supplied to the heater based on the measured output voltage.

10. The device according to any preceding claim, comprising the ambient temperature sensor connected to the controller and wherein the at least one function includes measuring an ambient temperature for the device.

11. The device according to claim 10, wherein the at least one function includes controlling a power supplied to the heater based on the measured ambient temperature.

12. The device according to any preceding claim, wherein the at least one function includes making a determination of the presence of a user input command to an input of a user interface of the device.

13. The device according to any preceding claim, wherein the at least one function includes updating an output of a user interface of the device.

14. The device according to any preceding claim, wherein the at least one function includes determining a puff status of a puff sensing means of the device.

15. The device according to any preceding claim, wherein the at least one function includes making a determination of a charging status of a power supply of the device.

16. The device according to any preceding claim, wherein the controller includes a microcontroller or a microprocessor.
